# EUROPEAN PATENT SPECIFICATION

(11) **EP 1 448 517 B1**
(45) Date of publication and mention of the grant of the patent: **18.04.2012**
(21) Application number: 02788903.9
(22) Date of filing: 30.10.2002
(51) Int. Cl.: C07C 319/14

(54) **METHOD OF PREPARING 4-CHLORO-3-METHYL-2-BUTENYLPHENYL SULFIDE, DI(4-CHLORO-3-METHYL-2-BUTENYL)SULFIDE AND DI(4-CHLORO-3-METHYL-2-BUTENYL)SULFONE FOR SYNTHESIS OF NATURAL CAROTENOID PRODUCTS**
VERFAHREN ZUR HERSTELLUNG VON 4-CHLOR-3-METHYL-2-BUTENYL-PHENYLSULFID, DI(4-CHLOR-3-METHYL-2-BUTENYL)SULFID UND DI(4-CHLOR-3-METHYL-2-BUTENYL)SULFON ZUR SYNTHESE VON NATÜRLICHEN CAROTINOIDPRODUKTEN
PROCEDE DE PREPARATION DE 4-CHLORO-3-METHYL-2-BUTENYLPHENYL SULFURE, DE DI(4-CHLORO-3-METHYL-2-BUTENYL) SULFURE, ET DE DI(4-CHLORO-3-METHYL-2-BUTENYL) SULFONE POUR LA SYNTHESE DE CAROTENOIDES NATURELS

(30) Priority: 31.10.2001 KR 2001067305
(43) Date of publication of application: 25.08.2004
(73) Proprietor: SK Innovation Co., Ltd., Jongro-gu Seoul 110-110 (KR); Koo, Sangho, Seoul 135-836 (KR)
(72) Inventor: KOO, Sangho, 135-836 Seoul (KR); YANG, Jae-Deuk, 142-875 Seoul (KR); KIM, Jeong-Soo, 305-728 Daejeon (KR); LEE, Sijoon, 305-762 Daejeon (KR); PARK, Minsoo, 302-740 Daejeon (KR)
(74) Representative: Moinas, Michel
(86) International application number: PCT/KR2002/002025
(87) International publication number: WO 2003/037854

(56) References cited:
- JP-A- 2000 063 351
- KR-A- 2001 086 682
- US-B1- 6 297 416
- DATABASE CA [Online] CHEMICAL ABSTRACTS SERVICE, COLUMBUS, OHIO, US; TSVETKOVA, TATYANA M. ET AL: "Process for preparing 4-halo-3-methyl-3-hydroxybutenes" XP002362807 retrieved from STN Database accession no. 1996:313595 & RU 2 047 591 C1 (BELGORODSKIJ FILIAL VSESOYUZNOGO NAUCHNO-ISSLEDOVATELSKOGO VITAMINNOGO) 10 November 1995 (1995-11-10)
- QUINKERT, GERHARD ET AL: "Totalsynthese des Pseudoguaianolids (+)-Confertin" LIEBIGS ANNALEN DER CHEMIE , (4), 283-315 CODEN: LACHDL; ISSN: 0170-2041, 1988, XP008057441
- CHOI, HOJIN ET AL: "Diallylic Sulfides as Key Structures for Carotenoid Syntheses" JOURNAL OF ORGANIC CHEMISTRY , 64(21), 8051-8053 CODEN: JOCEAH; ISSN: 0022-3263, 1999, XP002362586
- DATABASE CA [Online] CHEMICAL ABSTRACTS SERVICE, COLUMBUS, OHIO, US; MUSORIN, G. K. ET AL: "Preparation of di(propen-1-yl)sulfone by hydrogen peroxide oxidation of the sulfide" XP000250484 retrieved from STN Database accession no. 1992:40906 & SU 1 641 809 A1 (IRKUTSK INSTITUTE OF ORGANIC CHEMISTRY, USSR) 15 April 1991 (1991-04-15)

## Description

### TECHNICAL FIELD

The present invention pertains to preparation methods of the intermediates required for synthesis of carotenoid products. More specifically, the present invention is directed to, upon synthesis of natural carotenoid products by the Julia reaction using sulfone compounds for the formation of double bonds, the preparation methods of 4-chloro-3-methyl-2-butenyl phenyl sulfide represented by the following Formula 2 as a C10-unit compound for providing a central triene moiety:

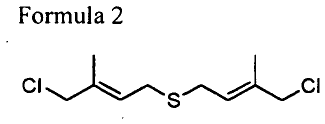

### PRIOR ART

Typically represented by beta-carotene, lycopene and astaxanthin, carotenoid compounds represented by the following Formula 4 act as a precursor of vitamin A importantly affecting differentiation and growth of living tissues:

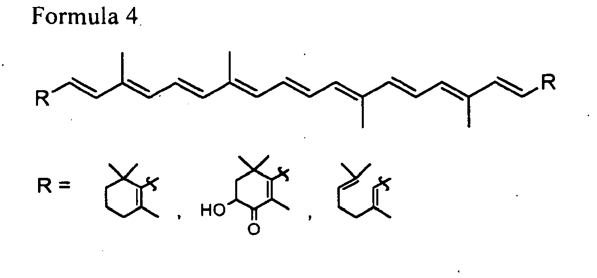

Further, such compounds are used as a food coloring material harmless to the human body, and have been used for promoting nutrition and supporting health due to the prophylaxis effects on cancers. Since the carotene compound is extracted in very small amounts from green-yellow vegetables, there has been research into the development of organic synthetic methods for carotenoid compounds. Such efforts have been made for quite a while.

Organic synthetic methods have been developed for several of important carotenoid compounds among hundreds of natural carotenoids. Representative syntheses are classified into three methods depending on the manner of forming double bonds, for instance, a Roche method using acetylide, a BASF AG method using the Wittig reaction, and the Julia reaction using sulfone compounds. Specifically, the Roche method forms double bonds by partial hydrogenation of triple bonds after a desired carbon backbone is obtained from acetylide. However, this method is disadvantageous in terms of many preparation steps, and formation of many cis double bonds having relatively low biochemical activity. Alternatively, the BASF AG method produces carotenoid compounds having double bonds with the use of the Wittig reaction, and has advantages in terms of relatively simple and efficient preparation process. The above method, however, suffers from drawbacks, such as difficulties in the preparation of a Wittig salt containing polyene and in the treatment of reaction by-products. Finally, the Julia method of synthesizing carotenoid compounds through the formation of double bonds using sulfone compounds, developed by the present inventors, has advantages of utilizing relatively stable sulfone compounds as synthetic intermediates, a simple reaction process and easy treatment of by-products, and is recognized as an excellent synthetic method *(*J. Org. Chem. 1999, 64, 8051-8053; Angew. Chem. Int. Ed. 2001, 40, 3627-3629; Korean Patent Laid-open Publication 2002-59202).

The method of synthesizing carotenoid compounds by the Julia reaction using sulfone compound is characterized in that, as shown in the following Reaction Scheme 1, two molecules of C15 sulfone compounds are coupled with di(4-chloro-3-methyl-2-butenyl) sulfide as a C10-unit represented by the above Formula 2 to form a fundamental carbon backbone required for the synthesis of a carotenoid compound, which is subjected to Ramberg-Backlund reaction to form a central triene moiety of the carbon backbone, which is then desulfonated while forming double bonds, thereby yielding a carotenoid compound:

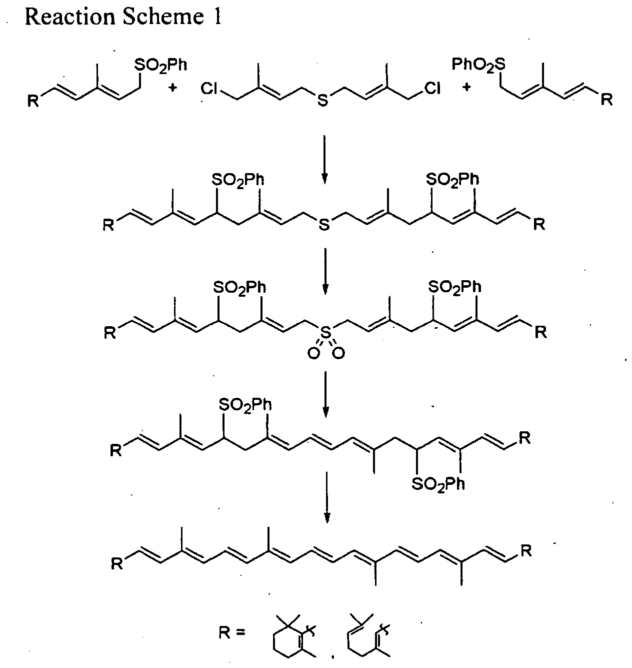

Suitable for use in the synthesis of lycopene following the above procedure, the C15 sulfone compound was afforded by the coupling reaction of easily producible geranyl sulfone as a C10-unit compound with 4-halo-3-methyl-2-butenyl phenyl sulfide represented by the following Formula 1 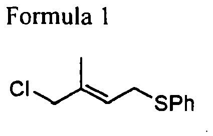 as a C5 isoprenyl unit, as shown in the following Reaction Scheme 2 *(*Angew. Chem. Int. Ed. 2001, 40, 3627-3629; Korean Patent Laid-open Publication No. 2002-59202):

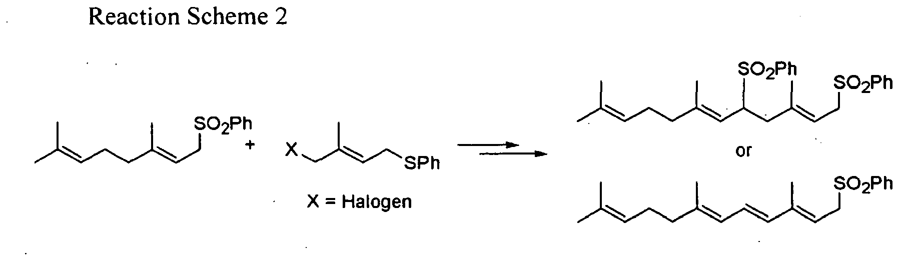

As in the above Reaction Schemes 1 and 2, 4-chloro-3-methyl-2-butenyl phenyl sulfide (Formula 1) as a C5-unit and di(4-chloro-3-methyl-2-butenyl) sulfide (Formula 2) as a C10-unit, which are developed by the present inventors and efficiently used for the synthesis of carotenoid compounds, are afforded according to procedures of the following Reaction Scheme 3:

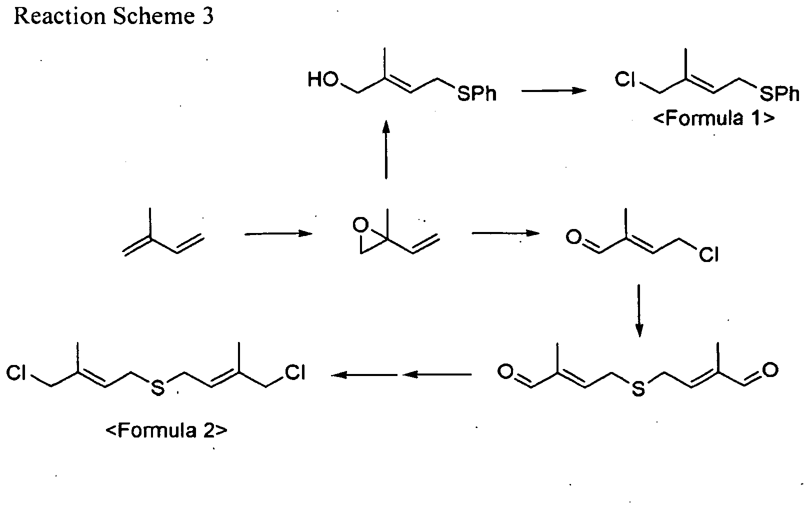

In the Reaction Scheme 3, isoprene is oxidized to isoprene monoxide, which is reacted with benzenethiol (PhSH) in the presence of a Cu(I) catalyst to perform a ring-opening reaction, giving a hydroxy-sulfide compound, which is subjected to halogenation of the hydroxyl group, thereby yielding 4-chloro-3-methyl-2-butenyl phenyl sulfide (Formula 1) *(*Angew. Chem. Int. Ed. 2001, 40, 3627-3629). Separately, isoprene monoxide is subjected to a ring-opening reaction using cupric chloride (CuCl₂) to form chloroaldehyde, two molecules of which are coupled with sodium sulfide (Na₂S) to obtain a C10 dialdehyde compound, which then forms the corresponding alcohol through aldehyde-reduction, after which the alcohol compound is subjected to chlorination, thereby giving di(4-chloro-3-methyl-2-butenyl) sulfide (Formula 2) as a C10-unit compound *(*J. Org. Chem. 1999, 64, 8051-8053).

Large scale preparations of 4-chloro-3-methyl-2-butenyl phenyl sulfide. (Formula 1) as the C5-unit compound and di(4-chloro-3-methyl-2-butenyl) sulfide (Formula 2) as the C10-unit compound according to the procedures of the above Reaction Scheme 3, however, suffer from the following disadvantages: first, following the ring-opening reaction of isoprene monoxide using benzenethiol (PhSH), phenyldisulfide (PhS-SPh) which is an oxide of benzenethiol is produced in an appreciable amount, and silica gel column chromatography should be additionally performed to remove the above oxide; second, the chloroaldehyde compound resulting from the ring-opening reaction of isoprene monoxide using cupric chloride is produced in low yield, and the dechlorinated aldehyde compound is also produced as a side-product, and it is difficult to treat the reaction mixture and a by-product of cuprous chloride in the work-up process; third, chlorination of the alcohol obtained by reduction of C10 dialdehyde is problematic in treating the reaction mixture and removing the by-products.

With the aim of synthesizing carotenoid products using the compounds represented by the Formulas 1 and 2 on a large scale, it is required to develop easy and efficient synthetic methods of the compounds represented by the Formulas 1 and 2 while overcoming the above problems. In addition, upon synthesis of a carotenoid compound according to the Reaction Scheme 1, selective oxidation of the sulfide moiety at the center to the sulfone group is difficult to perform due to many double bonds present in the carbon backbone. Thus, the problems related to achieving oxidation should be resolved.

### DISCLOSURE OF THE INVENTION

It is, therefore, an object of the present invention to solve the problems encountered in the prior art and to provide a method of preparing di(4-chloro-3-methyl-2-butenyl) sulfide represented by the Formula 2 as a C10-unit compound.

According to an embodiment of the present invention, there is provided a preparation method of di(4-chloro-3-methyl-2-butenyl) sulfide as a C10-unit compound (Formula 2) comprising the steps represented by the following Reaction Scheme 5, the method comprising (1) synthesizing 1-chloro-2-methyl-3-buten-2-ol (A) as a chlorohydrin compound from isoprene; (2) synthesizing 4-halo-1-chloro-2-methyl-2-butene (B) by halogenation (bromination or iodination) involving allylic rearrangement of the allylic alcohol of 1-chloro-2-methyl-3-buten-2-ol (A); and (3') coupling two molecules of 4-halo-1-chloro-2-methyl-2-butene (B) with sodium sulfide (Na₂S) while selectively leaving X with higher reactivity than Cl:

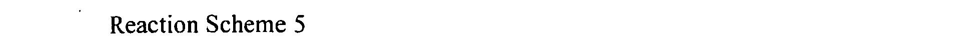

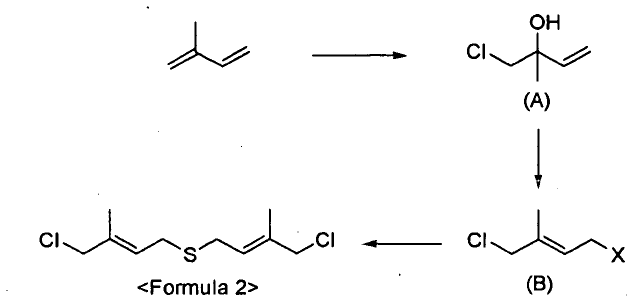

Where X represents Br or I.

### BEST MODES FOR CARRYING OUT THE INVENTION

Based on the present invention aiming at preparation of the intermediates required for synthesis of natural carotenoid products, there are provided more effective and practical preparation methods which have overcome the problems in conventional synthesis of di(4-chloro-3-methyl-2-butenyl) sulfide (Formula 2) as a C10-unit compound to solve the problems of selective oxidation of sulfides to sulfones in the presence of polyene, and a preparation method thereof.

As shown in the following Reaction Scheme 5, di(4-chloro-3-methyl-2-butenyl) sulfide (Formula 2) as a C10-unit compound is obtained by (1) synthesizing 1-chloro-2-methyl-3-buten-2-ol (A) by formation of chlorohydrin from isoprene, (2) synthesizing 4-halo-1-chloro-2-methyl-2-butene (B) by halogenation (bromination or iodination) through allylic rearrangement of the allylic alcohol of 1-chloro-2-methyl-3-buten-2-ol (A), and (3') coupling two molecules of 4-halo-1-chloro-2-methyl-2-butene (B) with sodium sulfide (Na₂S) while selectively leaving X with higher reactivity than Cl:

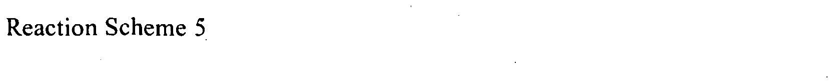

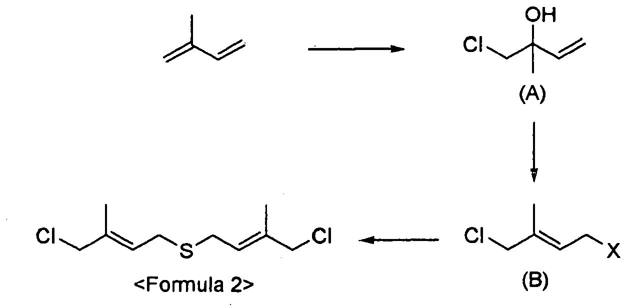

Where, X represents Br or I.

At the step (1) in the above reactions, the formation procedure of the chlorohydrin compound (A) from isoprene is disclosed in British Patent No. 978,892 (1964) concerning a method of blowing CO₂ into an aqueous solution of NaOCl, and in Russian Patent No. 2,047,591 (1995) regarding a method of reacting isoprene with NCS (N-chlorosuccinimide) using a polar solvent containing water at room temperature (20-25 °C). The former method is more economical due to the use of an inexpensive reagent, while the latter method is excellent in terms of high efficiency of reaction.

In the present invention, a polar solvent is not used, and a slight excess of isoprene (1.2 equivalents) is reacted with NCS (1 equivalent) in the presence of water at 30-100 °C, to obtain the chlorohydrin compound (A) having high yield and high purity to the extent of not requiring an additional purification procedure.

The halogenation involving allylic rearrangement of the step (2) in the above Reaction Scheme 5, may use PBr₃, Br₂/P, Br₂/PPh₃, NBS/PPh₃, I₂/PPh₃, NIS/PPh₃, etc. In particular, when 0.4 equivalents of PBr₃ are used based on the chlorohydrin compound (A) in the presence of a copper (I) salt catalyst including CuI, CuBr, CuCl and CuCN, trans double bonds (8:1 or more) are mainly formed. Further, no halogenated compound without allylic rearrangement, that is to say, 2-bromo-1-chloro-2-methyl-3-butene are produced at all in the presence of the copper (I) salt.

At the step (3') in the above Reaction Scheme 5, making use of a reactivity difference between the two halogen substituents Cl and X (Br or I) of the allylic dihalo compound (B) obtained at the step (2) toward a Na₂S nucleophile, X is selectively left while inducing a coupling reaction of the dihalo compound (B) with S²⁻, to synthesize di(4-chloro-3-methyl-2-butenyl) sulfide as represented by the Formula 2. As such, 2 equivalents of the compound (B) should be used on the basis of 1 equivalent of Na₂S nucleophile. As for the two double bonds of the C10-uni compound, a ratio of a trans-trans form to a trans-cis form is 4:1 or more. It is preferred that the reaction is performed at a low temperature (0 °C or lower) in order to increase selectivity of the reaction, and THF or lower alcohol is used as a reaction solvent.

When carotenoid compounds are typically synthesized using the diallylic sulfide of the Formula 2 (Reaction Scheme 1), undesired non-selective oxidation of a sulfide group occurs due to many conjugated double bonds present in the coupled C40 sulfide compound.

As such, double bonds and allylic chloride in the compound of the Formula 2 are stable to oxidation conditions.

Having generally described this invention, a further understanding can be obtained by reference to certain specific examples which are provided herein for purposes of illustration only and are not intended to be limiting unless otherwise specified.

### EXAMPLE 1

### Synthesis of 1-Chloro-2-methyl-3-buten-2-ol (A)

To a mixture of isoprene (24ml, 0.24mol) and water (100ml) was added NCS (*N-*chlorosuccinimide, 26.7g, 0.20mol), after which the reaction mixture was maintained at 60 °C for 24 hours and cooled to room temperature. A resultant material was extracted with diethyl ether, washed with water, dried over anhydrous sodium sulfate, filtered and concentrated under reduced pressure, to give 1-chloro-2-methyl-3-buten-2-ol (18.8g, 0.156mol, 78%) which was pure to the extent of not requiring further purification.
¹H NMR δ 1.38 (3H, s), 2.17 (1H, br s), 3.53 (1H, d of A of ABq, *J_{d} =* 11.0, *J_{AB}* = 12.1 Hz), 3.57 (1H, d of B of ABq, *J_{d}* = 11.0, *J_{AB}* = 12.1 Hz), 5.21 (1H, dd, *J* = 10.7, 1.0 Hz), 5.38 (1H, dd, *J* = 17.3, 1.0 Hz), 5.92 (1H, dd, *J* = 17.3, 10.7 Hz) ppm.
¹³C NMR δ 25.3, 54.0, 72.5, 114.7, 141.9 ppm

### EXAMPLE 2

### Synthesis of 4-Bromo-1-chloro-2-methyl-2-butene, (B)

1-Chloro-2-methyl-3-buten-2-ol (23.5g, 0.195 mol) obtained in the above example 1 was dissolved in anhydrous diethyl ether (100ml), to which CuBr (559mg, 3.9mmol) was added, and PBr₃ (6.5ml, 78.1 mmol) was cautiously further added at 0 °C. The reaction mixture was stirred at room temperature for 2 hours, diluted with 100ml of diethyl ether, washed with water, dried over anhydrous sodium sulfate, filtered and concentrated under reduced pressure, to give 4-bromo-1-chloro-2-methyl-2-butene (28.2g, 0.146mol, 75%). As such, a ratio of trans to cis double bonds was 8:1 or more. Further, 2-bromo-1-chloro-2-methyl-3-butene by bromination without allylic rearrangement was not produced at all.
trans: ¹H NMR δ 1.85 (3H, s), 3.98 (2H, dd, *J* = 8.3, 0.9Hz), 4.03 (2H, s), 5.87 (1H, dt, *J_{d}* = 0.9, *Jₜ* = 8.3 Hz) ppm
¹³C NMR δ 14.1, 27.3, 50.6, 125.3, 137.8 ppm
cis: ¹H NMR δ 1.92 (3H, s), 4.00 (2H, dd, *J* = 8.3, 0.7Hz), 4.10 (2H, s), 5.73 (1H, dt, *J_{d}* = 0.7, *Jₜ* = 8.3 Hz) ppm

### EXAMPLE 3

### Synthesis of Di(4-chloro-3-methyl-2-butenyl) Sulfide Represented By Formula 2

4-Bromo-1-chloro-2-methyl-2-butene (15.6g, 80.7mmol) obtained in the above example 2 was dissolved in tetrahydrofuran (100ml), to which Na₂S (6.76g, 40.3mmol) was added at 0 °C. The reaction mixture was stirred at 0 °C for 6 hours, added with water, extracted with diethyl ether, washed with water, dried over anhydrous sodium sulfate, filtered and concentrated under reduced pressure. A crude product was purified by silica gel chromatography, to give di(4-chloro-3-methyl-2-butenyl) sulfide (7.83g, 32.7mmol, 81 %). As such, in a double bond structure, a ratio of a trans-trans compound to a trans-cis compound was 4:1 or more.
trans-trans: ¹H NMR δ 1.78 (3H, s), 3.10 (2H, d, *J* = 7.6 Hz), 4.03 (2H, s), 5.62 (1H, t, *J* = 7.6 Hz) ppm
trans-cis: ¹H NMR δ 1.83 (3H, s), 3.12 (2H, d, *J* = 8.1 Hz), 4.01 (2H, s), 5.44 (1H, t, *J* = 8.1 Hz) ppm

### INDUSTRIAL APPLICABILITY

As described above, in the synthesis of natural carotenoid products using sulfone compounds, the inventive preparation method of di(4-chloro-3-methyl-2-butenyl) sulfide represented by the Formula 2 as a C10-unit compound is advantageous in terms of high preparation efficiency due to a relatively simple process, and suitability for large scale preparation, while solving the problems in conventional synthetic methods. Thereby, through the methods of the present invention, various carotenoid products can be efficiently and economically prepared.

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

## Claims

1. A method of preparing di(4-chloro-3-methyl-2-butenyl) sulfide comprising the steps represented by the following Reaction Scheme 5,
the method comprising the steps of:
(1) synthesizing 1-chloro-2-methyl-3-buten-2-ol (A) as a chlorohydrin compound from isoprene;
(2) synthesizing 4-halo-1-chloro-2-methyl-2-butene (B) by halogenation (bromination or iodination) involving allylic rearrangement of the allylic alcohol of 1-chloro-2-methyl-3-buten-2-ol (A); and
(3') coupling two molecules of 4-halo-1-chloro-2-methyl-2-butene (B) with sodium sulfide (Na₂S) while selectively leaving X, thereby affording di(4-chloro-3-methyl-2-butenyl) sulfide represented by the Formula 2 as a C10-unit compound: 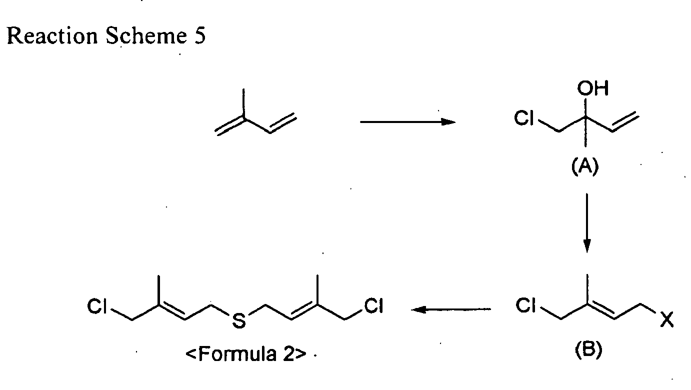
Where X represents Br or I.

2. The method as defined in claim 1, wherein the step (1) is performed through reaction of isoprene and N-chlorosuccinimide in water at 30-100°C.

3. The method as defined in claim 1 or 2, wherein the step (2) is performed using PBr₃ in the presence of a copper (I) salt catalyst.

4. The method as defined in any one of claims 1 to 3, wherein the step (3') is performed using THF or lower alcohol solvent at 0°C or lower.

## Patentansprüche

1. Verfahren zur Herstellung von Di-(4-Chlor-3-methyl-2-butenyl)-sulfid, umfassend die Schritte, die durch das folgende Reaktionsschema 5 dargestellt sind, wobei das Verfahren die Schritte umfasst:
(1) Synthetisieren von 1-Chlor-2-methyl-3-buten-2-ol (A) als eine Chlorhydrin-Verbindung aus Isopren;
(2) Synthetisieren von 4-Halo-1-Chlor-2-methyl-2-buten (B) durch Halogenierung (Bromierung oder Jodierung), das eine Allylumlagerung des Allylalkohols von 1-Chlor-2-methyl-3-buten-2-ol (A) beinhaltet; und
(3') Verknüpfung zweier Moleküle von 4-Halo-1-Chlor-2-methyl-2-buten (B) mit Natriumsulfid (Na₂S), indem X selektiv abgeht, wodurch Di-(4-Chlor-3-methyl-2-butenyl)-sulfid, das durch die Formel 2 dargestellt ist, als eine Verbindung mit C10-Einheit erzeugt wird: 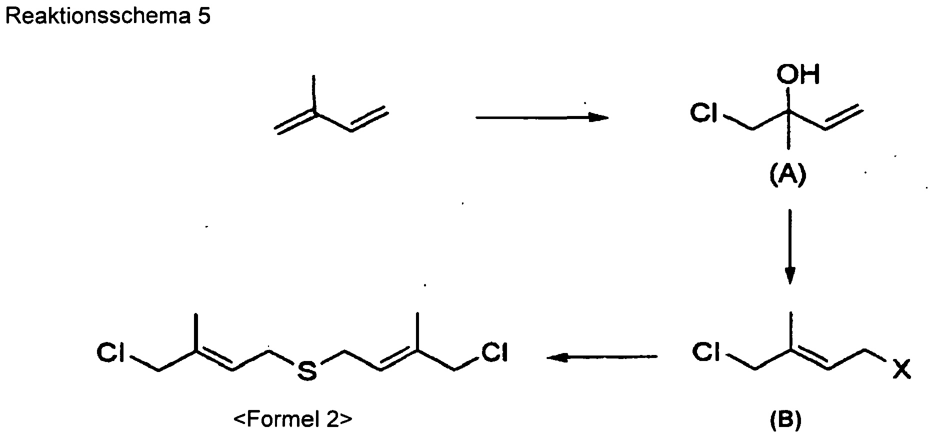
worin X Br oder I darstellt.

2. Verfahren nach Anspruch 1, wobei der Schritt (1) durch die Reaktion von Isopren und N-Chlorsuccinimid in Wasser bei 30-100°C durchgeführt wird.

3. Verfahren nach Anspruch 1 oder 2, wobei der Schritt (2) unter Verwendung von PBr₃ in der Anwesenheit von einem Kupfer(I)-Salz-Katalysator durchgeführt wird.

4. Verfahren nach einem der Ansprüche 1 bis 3, wobei der Schritt (3') unter Verwendung von THF oder einem niederem Alkohol-Lösungsmittel bei 0°C oder niedriger durchgeführt wird.

## Revendications

1. Une méthode de préparation du sulfure de di(4-chloro-3-méthyl-2-butényle) comprenant les étapes représentées par le schéma réactionnel 5 suivant,
la méthode comprenant les étapes de :
(1) synthèse du 1-chloro-2-méthyl-3-butèn-2-ol (A) comme un composé chlorhydrine à partir de l'isoprène ;
(2) synthèse du 4-halo-1-chloro-2-méthyl-2-butène (B) par halogénation (bromuration ou ioduration) impliquant un réarrangement allylique de l'alcool allylique de 1-chloro-2-méthyl-3-butèn-2-ol (A) ; et
(3') couplage de deux molécules de 4-halo-1-chloro-2-méthyl-2-butène (B) avec du sulfure de sodium (Na₂S) tout en éliminant sélectivement X, aboutissant ainsi au sulfure de di(4-chloro-3-méthyl-2-butényl) représenté par la formule 2 comme un composé ayant une unité C10. 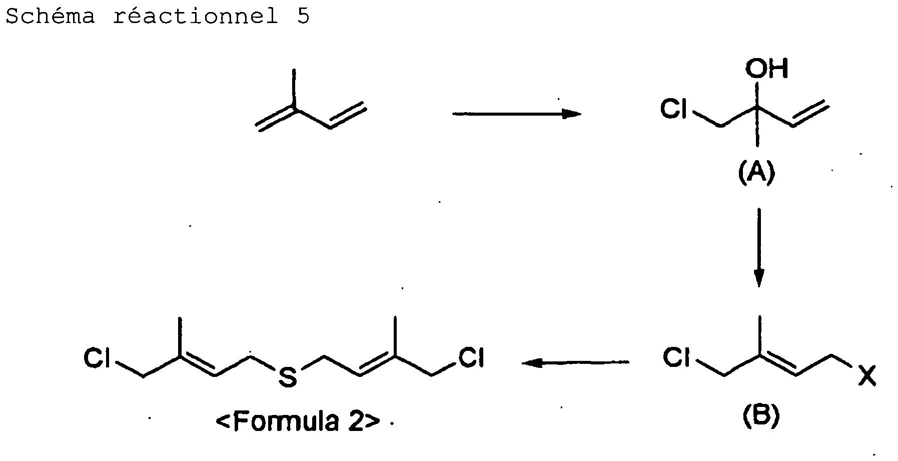
dans laquelle X représente Br ou I.

2. La méthode comme définie dans la revendication 1, dans laquelle l'étape (1) est réalisée par réaction d'isoprène et de N-chlorosuccinimide dans de l'eau à 30-100°C.

3. La méthode comme définie dans la revendication 1 ou 2, dans laquelle l'étape (2) est réalisée en utilisant du PBr₃ en présence d'un catalyseur de sel de cuivre (I).

4. La méthode comme définie dans n'importe laquelle des revendications 1 à 3, dans laquelle l'étape (3') est réalisée en utilisant du THF ou un solvant alcoolique inférieur, à 0°C ou moins.
